# EUROPEAN PATENT APPLICATION

(11) **EP 3 403 603 A1**
(43) Date of publication of application: **21.11.2018**
(21) Application number: 17171157.5
(22) Date of filing: 15.05.2017
(51) Int. Cl.: A61B 17/34, A61B 90/00, A61M 37/00, A61N 5/10

(54) **BRACHYTHERAPY SET**

(71) Applicant: Contract Medical International GmbH, 01277 Dresden (DE)
(72) Inventor: HURTAK, Wenzel, 83310 Cogolin (FR); WIESNER, Thomas, 01454 Wachau (DE); MATTHES, Martin, 01809 Heidenau (DE); THALWITZER, Jörg, 08066 Zwickau (DE)
(74) Representative: Simandi, Claus

(57) **Abstract**

Tools for brachytherapy providing an introducer sheath and an afterload catheter with particular marks and gratings that allow for a more precise and safe positioning of the tools and the load of the afterload catheter.

## Description

The present invention relates to methods and means for brachytherapy and provides specifically designed set of brachytherapy tools which, in combination with each other, allows for improved and more safe brachytherapy use, in particular an afterload catheter with particular marks and gratings coinciding with an introducer sheath and puncture needle, that allow for a more precise and safe positioning of the tools and the load of the afterload catheter.

Brachytherapy is an internal radiation therapy for a so-called short distance therapy, wherein an enclosed radioactive source is precisely positioned within or in close proximity to a tissue to be treated, e.g. tumor, within the animal or human body. Brachytherapy is commonly used for the treatment of cancer of cervix, prostate, breast or skin. The application of known brachytherapy tools, bears high risk of iatrogenic complications and/or less therapeutic effect.

For the precise placement of a load, in particular a radiation source for use in brachytherapy, brachytherapy tools are provided and utilized in a particular fashion: in an initial step the skin and tissue of the body above the area of interest is punctured by a puncture needle having a cylindrical shaft and a cutting blade tip. The puncture needle is advanced onto the region of interest, for example a tissue or organ to be treated with the load, thereby cutting a channel from the skin onto the area of interest. The needle can now be retracted and a tubular introducer sheath which is fitted over a dilator rod is introduced and protruded into the cut channel. The dilator is then retracted, leaving the tubular introducer sheath in place. In one or more further steps, a so-called afterload catheter can be repeatedly introduced into the introducer sheath that left in place at the cut channel. The afterload catheter may have a container that may carry the load, for example and in particular, a radiation source. The loaded afterload catheter may be left in place within the channel during treatment and is then retracted.

One particular problem of brachytherapy tools known in the art is the difficulty to position the load borne by the afterload catheter precisely at or into the region of interest, i.e. at the end of the originally produced channel, cut by the puncture needle. That may lead to a less effective therapy, because the load may not be properly positioned at the tissue. Even iatrogenic complications during treatment may occur, in particular if the afterload catheter and/or the introduced sheath/dilator assembly is protruded to deep, i.e. deeper than the cut channel. For example, the tools' tip might be progressed into deeper areas and may damage deeper tissue, vessels or organs.

This technical problem is primarily solved by the provision of a newly designed afterload catheter of or in a brachytherapy set of tools, according to claim 1. The afterload catheter of the invention particularly comprises a visual exit mark, which in accordance with the present invention, is positioned on the shaft of the afterload catheter such, that it visibly coincides with the proximal cap of the introducer sheath when the tip, that is in particular the load, of the afterload catheter is at the distal opening of the introducer sheath. That is, the exit mark on the shaft of the afterload catheter is visibly positioned directly on the proximal inlet of the introducer sheath as soon as or when the tip of the afterload catheter is at the aimed position, that is on the open tip of the introducer sheath. By that, the applying person, i.e. the surgeon, can easily recognize that the load provided at the tip of the afterload catheter is precisely at the aimed position within the tissue as soon as the exit mark on the shaft of the afterload catheter coincides with the (proximal) inlet of the introducer sheath.

The "aimed position" or "region of interest" is primarily the end of the cut channel, i.e. a tip of the puncture needle., it is, however, not limited thereto: There may be one or more "regions of interest" along the way of the cut channel, i.e. at predefined intermittent positions along the originally produced channel.

The specifically positioned visual exit mark on the afterload catheter according to the present invention allows for safe and precise positioning of the load at the tip of the afterload catheter without the need of any further specific positioning tools. This is of particular importance and benefit in the utilization of the afterload catheter, which, in contrast to the needle and introducer sheath/dilator assembly, has to be applied under less controllable conditions, for instance in radiation therapy or other critical diagnostic or therapeutic applications. While the initial puncture needle and consecutively the introducer sheaths may be positioned by known means of surgical online control, such as ultrasound, X-ray or the like, or, alternatively, or in addition, by means of robotic aided or robotic performed surgery, in the first place. This is of particular importance when repeated positioning or re-loading of the afterload catheter is required, for example during the course of clinical therapy or in various instances of diagnostic measurements or probing.

In a preferred embodiment, the afterload catheter further comprises on its shaft at least one further visual mark or grating which is positioned further proximal to said exit mark. This advantageously allows for controlled advancement of the afterload catheter tip yet beyond the original position, i.e. beyond the open tip of the introducer sheath as a reference point for controlled and thus safe advancing of the afterload catheter beyond that reference point. In a preferred embodiment, three markers proximal of the initial exit mark are present, at 1 cm distance from each other to mark catheter advancement beyond the sheath tip.

For an even better positioning of the tubular introducer sheath at the site of interest, the present invention preferably and in addition provides an improved introducer sheath that comprises a visual scale or creating at least at the cylindrical or tubular part of the introducer sheath. This particular scale of grating is meant to coincide or correspond with a respective grating or visual depth markings at the respective puncture needle that cuts the channel onto the site of interest within a tissue. By use of these corresponding or coinciding depth markings on both, the puncture needle and the introducer sheath, this particular embodiment of the present invention advantageously allows for the precise introduction of the introducer sheath onto the very same position, i.e. depth, where the puncture needle has been placed before to cut the channel in the first place. In the simplest variant, the puncture needle in accordance with a preferred embodiment of the present invention, carries a visual scale on its outside and, for example, the outer skin of the human or animal body is used as a reference point, the reading of the scale of the finally positioned puncture needle shall correspond to the reading of the corresponding visual scale or creating a provided at the outside of a preferred embodiment of the introducer sheath. This ensures that the open distal tip of the introducer sheath can be positioned at same place, up to which the channel has been cut by the puncture needle. The principle of the invention relies on the finding that Printed depth markings on the sheath, coinciding with puncture needle depth scale helps to significantly reduce risk of mistakes in placing the sheath tip to exactly the same depth as when the puncture needle was positioned.

The combination of the particular tools in accordance with a preferred embodiment of the present invention, i.e., the afterload catheter carrying a visual exit mark meant to be visibly inside with the proximal cap of the introducer sheath, secondly, the visual scale or grating of the outside of the introducer sheath which corresponds to the respective scale or marks on the outside of the puncture needle used for cutting the channel into the tissue represents a referencing system where the position of each tool is referenced to the other by means of respective scale, gratings or marks. In accordance therewith, a particular embodiment of the present invention provides a brachytherapy kit comprising an afterload catheter of the present invention and a tubular introducer sheath as disclosed herein, and optionally, but preferably, includes a puncture needle, preferably having visual depth markings designed to correspond with the scale or grating of the introducer sheath.

The visual marks, scales or gratings may be applied on the tools by standard means of marking. A preferred marking relies on laser printing. Yet another marking is based on ink printing, high contrast inks are preferred. It is required that the markings are such that they are visible with the naked eye to the applying person, i.e. surgeon. In further variants, fluorescent inks or dyes are used for the visual markings. In yet another variant, metal depositing methods such as plating or PVD and the like may be used to produce a visual mark, which is visible in X-ray, ultrasound or other tomography systems or may be detectable by electronic sensors for automated or assisted placement or positioning of the tools in particular the afterload catheter.

In a further and, in particular additional preferred embodiment, the kit further includes a dilator rod to be introduced or received within the tubular introducer sheath, particularly matched for snugly sliding within the sheath. The dilator rod of a preferred embodiment of the present invention comprises a distal section and a shaft which is characterized in that the distal section is tapered for enabling or facilitating the introduction of the introducer sheath into the channel cut by the puncture needle. According to this preferred embodiment of the invention, this distal section is tapered over a length of 10 mm at maximum, i.e., represents a relatively short tapered tip. It has been surprisingly found that this short tapering is sufficient to enable to facilitate the introduction of the introducer sheath onto the site of interest, but prevents additional tissue damage at that position within the tissue.

It has been found that this short, tapered tip reduces the distance between the dilator tip and tip of the introducer and reduces the damage to deeper lying tissue and the risk of bleeding complications or organ damage by a dilator tip which else would point too deep. The herein described elements and tools for brachytherapy are also useable for other applications and treatment which rely on the precise placement of a product, be it a pharmaceutical compound, an electrode, probe or sensor, within a defined region of the animal or human body. Thus, the elements and tools disclosed herein are not limited for use in brachytherapy but may have general applications in the medical field, in particular in diagnosis as well as in prophylactic or therapeutic treatment.

In a preferred embodiment, the tapering on the tip of the dilator has a length of 10 mm or less, preferably of 5 mm or less. The tip may be tapered to a guidewire, in particular a 0.035" guidewire. The smooth transition between dilator and a guidewire allows for optimal introduction and advancement into rigid (tumor) tissue.

In a preferred embodiment, the tapered distal section of the dilator, when properly received within the introducer sheath, extends beyond the distal opening of the introducer sheath, at least over the length of the tapering, i.e. preferably by 10 mm or less, preferably by 5 mm or less. This has been found to facilitate introduction of the introducer sheath with minimal risk of damage to the tissue.

The dilator may comprise a stiff but sufficiently flexible shaft material to be easily advanced in rigid (tumor) tissue. This particularly compensates for the less sharp, shorter, yet blunter tip.

Preferably, the outer diameter (OD) is matched to the inner diameter (ID) of the introducer sheath, and particularly the length of the dilator is matched to total length of the introducer sheath. In a particular embodiment, the distal tip or opening of the introducer sheath is also tapered, in particular bullet shaped. This allows for a tight fit with the tip of the dilator receivable or received within the sheath. A smooth transition between sheath and dilator allows for optimal introduction and advancement into rigid tissue such as tumor tissue.

The brachytherapy kit of the present invention may further include a guide wire. This may have a stiff distal end to secure dilator precise advancement to the needle puncture location, and a stiff proximal end to allow for easy frontloading of dilator & sheath.

The brachytherapy kit of the present invention may further include surgical suture with a preferably curved needle for attachment and fixation of sheath position.

The brachytherapy kit of the present invention may further include a suture clip with suture holes, specifically designed to snap and force clamp onto any position along the introducer sheath. The suture clip particularly allows for quick, easy and reliable fixation to any position at the sheath, and thus allows for fixation of sheath position by suturing the clip to the patient skin and at the same time for fixation of the afterload catheter through press force (clip).

The brachytherapy kit of the present invention may further include a dispensing tip, a small cannula with female luer, to facilitate injection of coagulant through the sheath, at the end of the intervention.

Further preferred design elements of the tools for brachytherapy disclosed herein contribute to the advantages and beneficial effects:
As regards the afterload catheter, this may preferably comprise or consist of a non-radiopaque polymer, and in particular is non-braided and non-coiled. This allows for optimal transparency for radiation. A preferred polymer material is resistant to gamma radiation (192 Ir, 380 keV) and exhibits low friction in connection with a moving radiation source.

In a preferred embodiment, the afterload catheter, which commonly is hollow, has a rounded, closed tip, and preferably an open proximal end. This allows for an easy insertion into the introducer sheath. This also allows a perfect seal between a radioactive load and the body tissue. The tip wall thickness is preferably 0.5 to 1.5 mm, more preferred 0.7 to 1.3 mm. Lumen diameter and lumen integrity throughout is critical for good gamma-source transfer. The hollow afterloader has low friction between inner wall and radiation source or load to be introduced. Thus the tolerance on free pathway in the lumen, measured from proximal opening may not exceed 0.5 mm

The afterload catheter may exhibit a stiffness which matches to the tissue application, preferably avoids kinking or wrinkling due to movement of the source and due to patient (breathing) movements and allows smooth guidance of load movement.

The afterload catheter preferably has a length of approx. 35 cm to be compatible with common afterloading systems and suitable for clinical situation (i.e. the depth of target tumor from the selected puncture site) Other lengths for other target organs or for e.g. obese patients are, of course, encompassed.

As regards the introducer sheath, this may preferably have an inner diameter of the shaft which corresponds to or is compatible to a dilator and/or afterload catheter to be received with the sheath, i.e. of Fr.6 size (approx. 1.5 mm). The preferred length is approx. 25 cm, an ideal length for percutaneous access to target tumor. The introducer sheath is preferably made of a radiopaque polymer with reduced radiopacity. The shaft is preferably non-braided. The sheath preferably exhibits modified, i.e. reduced radiopacity in comparison to known (endo-vascular) devices that are tailored for X-ray imaging (fluoroscopy). If has been found on such devices that under CT imaging many shadow images are showing. In particular, if a plurality of sheaths is placed in a tissue or tumor, the shadow images make it confusing to map exactly the individual positions in a 3-D visualization. The material and composition of the introducer sheath is thus preferably optimized for imaging under CT, to be well visible, without creating shadow images)

The introducer sheath may include a hemostasis valve with a standard silicone disc-valve to seal-off bleeding during device exchanges but while afterload catheter remains in place. The introducer sheath may be void of a flush-side port and/or of a 3-way-valve. This has been found to be not needed; its presence is adding weight and increasing the risk of sheath displacement from the tumor. In brachytherapy application, the presence of a sideport tubing and a 3-way valve increases the risk of getting snagged and the precisely placed sheath (with afterload catheter) being pulled out or being displaced.

The introducer sheath may be void of a suture ring near the valve, but rather foresees a movable suture clip, instead.

As regards the dilator to be placed in the introducer sheath, this may preferably have a lock- and easy disconnect feature on proximal end, i.e. in the connection between sheath to dilator. It has been found that known dilators for vascular applications typically "click" to the sheath, allowing the combined product to be inserted and to dilate the puncture, without the dilator backing out. Then using extra force, the "click" connection will give way and the dilator can be pulled back. This, however, may cause a shock and thus an abrupt displacement/dislocation of the sheath when the dilator is removed from the sheath after insertion into the cut channel. Thus the dilator and the introducer sheath have an interlocking assembly that avoids any "click" system, but rather relies on soft clamping and friction. In a further preferred embodiment, dilator and sheath are removably connectible via a shock-free connector/disconnector assembly. The dilator may thus comprise a "wing grip" that allow for non-disruptive removal of the dilator from the sheath, thereby minimizing the risk of accidental repositioning of the sheath during dilator removal.

In a preferred embodiment the brachytherapy tools of the invention include an integral introducer system, which is an intimate assembly of the dilator received in the fitted introducer sheath, with the dilator held or clamped to the sheath by means of a winged grip engaging or clamping a corresponding structure, e.g. rim or ridge, on the proximal inlet part of the introducer sheath described herein.

As regards the puncture needle, this may be comprised of a 2-pieced wire, i.e. a stylet placed in a surrounding cannula. The two parts can be locked together, e.g. via luer connection, to allow for precise puncture. After the puncture, the stylet may be unlocked by twisting, and taken out.

The puncture needle may comprise a stylet with a tri-bevel point for precise puncture. This is unlike a skived needle tip which may follow a curved trajectory on insertion.

A preferred variant of the guide wire of the brachytherapy kit is a short guide wire without a "floppy" tip, in particular a simple" non-coated nitinol wire having rounded blunt ends, but being void of a floppy tip "waiving" on the outside. This has been found to facilitate positioning of the dilator over the wire and to advance into the puncture cut channel. The wire is short, i.e. preferably of about 90 cm in length.

The figures show:

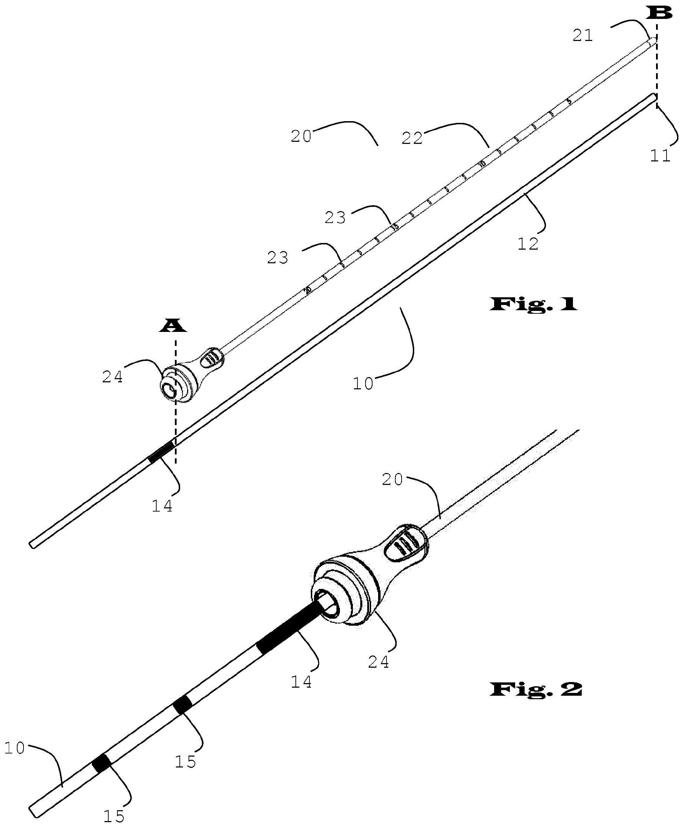

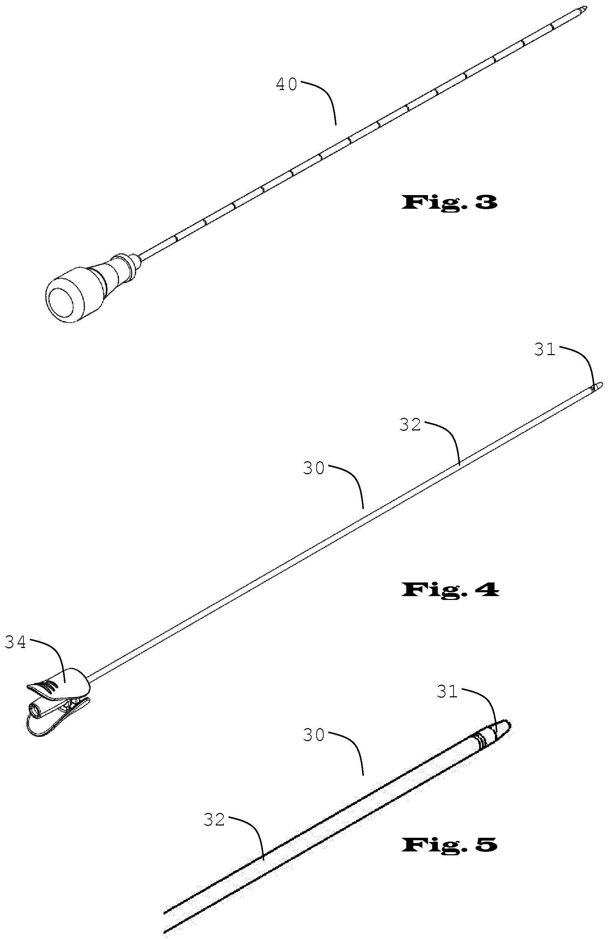

Figure 1 depicts a schematic view of the afterload catheter, (10) of the present invention having a rounded tip (11) and a cylindrical shaft (12) showing a visible exit mark (14) which is positioned such that when the afterload catheter (10) is received within the tubular introducer sheath (20). The exit mark (14) visibly coincides (line A) with the inlet (24) of the introducer sheath (20), if the proximal tip (11) of the afterload catheter is at the proximal opening (21) of the introducer sheath (line B), thus providing an easy and precise position matching system. Visual markings or grating (23) may also be present at least at the tubular cylindrical portion (22) of the introducer sheath (20) to correspond to respective visual markings or grating (43) of a puncture needle (40) depicted in Figure 3, thus providing an even advanced position matching system (see figure 3).
Figure 2 shows a detail from Figure 1 where the afterload catheter (10) is received within the introducer sheath (20) where the visual mark (14) of the afterload catheter (10) visibly coincides with the inlet (24) of the introducer sheath. Further, markings (15) are present which serve as depth markings for easy advancement of the afterload catheter (10) beyond the reference point, i.e. deeper into the tissue.
Figure 3 shows a puncture needle (40) for cutting the channel into the body tissue onto the region of interest. The needle (40) has markings (43) that correspond to markings (23) of the introducer sheath (20), which allow for advancing the introducer sheath (20) in the channel cut by the puncture needle (40) into the same depth, by using the respective corresponding markings (23, 43) as reference. The reference point may be the outer skin of the body at the site where the needle has been introduced. If the readings of markings (23) match with the reading of markings (43) at that reference point, the proximal tip opening (21) of introducer sheath (20) is at the same position as the tip of the puncture needle (40).

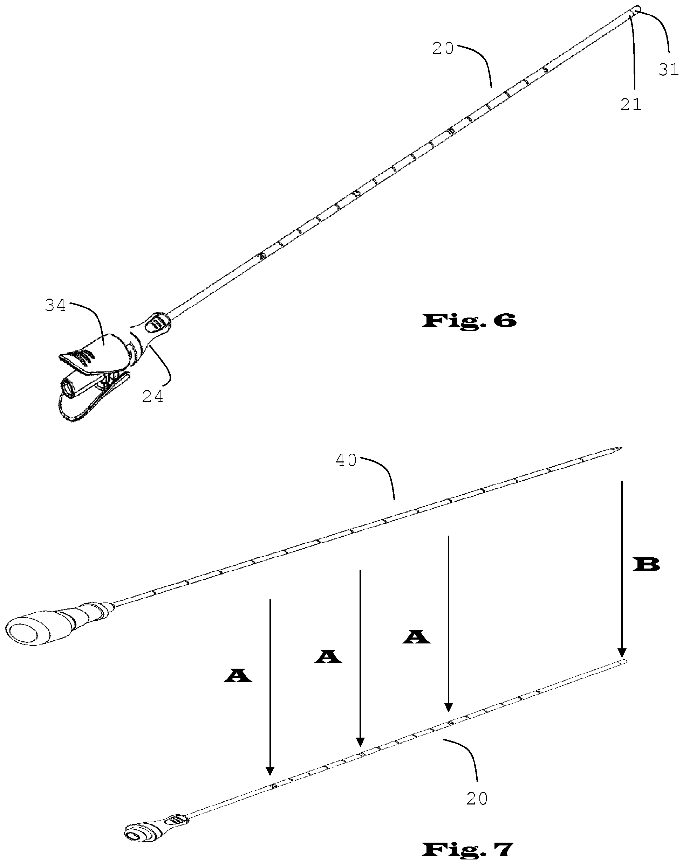

Figures 4, 5 and 6 show a dilator rod (30) to be received within the introducer sheath (20) for introducing the sheath (20) into the channel cut by the puncture needle (40). The dilator has a cylindrical shaft (32) that matches the ID of the sheath (20). The dilator (30) has a blunt short tip (31). The dilator (30) also comprises a wing grip (34) to removably clamp or engage with a respective structure on the proximal inlet (24) of the introducer sheath (20) to form a stable assembly of dilator (30) and sheath (20), i.e. an introducer system for introduction into the channel cut by needle (40). Figure 5 depicts a detail of the dilator (30) of figure 4 with tip (31). Figure 6 depicts the dilator (30) received within the introducer sheath of figure 1 to form an introducer system or assembly.
Figure 7 shows a typical arrangement of an embodiment of the invention, where depth markings (43) on the puncture needle (40) correspond to respective markings or scale (23) on the introducer sheath (20). This allows corresponding depth readings "A" on the sheath (20) with reference to each tip position "B", allowing precise placement or positioning of the introducer sheath (20), in particular as the introducer assembly depicted in figure 6, relative to the originally introduced puncture needle (40) that cut the channel.

### REFERENCE LIST

- 10: afterload catheter
- 11: catheter tip
- 12: cylindrical shaft
- 14: exit mark
- 15: mark or grating
- 20: introducer sheath
- 21: distal sheath opening
- 22: cylindrical part
- 23: markings
- 24: proximal inlet
- 30: dilator
- 31: tapered distal section
- 32: dilator shaft
- 34: wing grip
- 40: puncture needle
- 43: markings

## Claims

1. A brachytherapy afterload catheter (10), comprising a catheter tip (11), a cylindrical shaft (12) to be introduced and snuggly sliding in a tubular introducer sheath (20) having a distal opening (21), a cylindrical part (22) and a proximal inlet (24), **characterized in that**
a visual exit mark (14) is positioned on said shaft (12) of the afterload catheter (10) to visibly coincide with the proximal cap (24) of the introducer sheath (20) when said catheter tip (11) is at said distal opening (21) of the introducer sheath (20) .

2. The afterload catheter (10) of claim 1, further comprising on said shaft (12) at least one further mark or grating (15) proximal to said exit mark (14).

3. A brachytherapy kit, comprising:
- the afterload catheter (10) of claim 1 or 2,
- a tubular introducer sheath (20) to receive the afterload catheter, comprising a distal opening (21), a cylindrical part (22) and a proximal inlet (24).

4. The brachytherapy kit of claim 3, wherein at least the cylindrical part (22) of the introducer sheath (20) has a visual depth markings or scale (23), the kit further comprising:
- a puncture needle (40), **characterized in** having visual depth markings or scale (43) corresponding with visual depth markings or scale (23) of the introducer sheath (20).

5. The brachytherapy kit of claim 3 or 4, wherein the distal opening (21) of the introducer sheath (20), is tapered to allow for tight fit with the tip of a dilator (30) receivable within the sheath (20).

6. The brachytherapy kit of any one of claims 3 to 5, further comprising:
- a dilator rod (30) to be received in the tubular introducer sheath (20), comprising a distal section (31) and a shaft (32), **characterized in that** the distal section (31) is tapered over length of 10 mm or less.

7. The brachytherapy kit of claim 6, wherein the tapered distal section (31) of the dilator (30), when received within the sheath (20), extends beyond the distal opening (21) over the length of the tapering.

8. The brachytherapy kit of claim 6 or 7, wherein the dilator (30) is received within the sheath (20) and removably engaged with the sheath (20) via a wing clamp (34) engaging the proximal opening (24) of the sheath (20), thus forming an integral introducer assembly.

9. Afterload catheter according to claim 1 or 2 for use in Brachytherapy.

10. Brachytherapy kit according to any one of claims 3 to 8 for use in Brachytherapy.

11. Use of afterload catheter according to claim 1 or 2 for precise placement of a load carried by the afterload catheter at the position of the tip of an introducer sheath receiving the afterload catheter.

12. Use of brachytherapy kit according to any one of claims 3 to 8 for precise placement of a load carried by the afterload catheter (10) at the position of the tip (21) of an introducer sheath (20) receiving the afterload catheter (10).
